# EUROPEAN PATENT SPECIFICATION

(11) **EP 4 186 370 B1**
(45) Date of publication and mention of the grant of the patent: **01.11.2023**
(21) Application number: 21210909.4
(22) Date of filing: 26.11.2021
(51) Int. Cl.: A23C 19/055, A23L 5/44, A23C 19/05

(54) **CAROTENOID COLORING COMPOSITION FOR CHEESE PRODUCTS**
CAROTINOID-FARBSTOFFZUSAMMENSETZUNG FÜR KÄSEPRODUKTE
COMPOSITION COLORANTE À BASE DE CAROTÉNOÏDES POUR LES PRODUITS FROMAGERS

(43) Date of publication of application: 31.05.2023
(73) Proprietor: Oterra A/S, 2970 Hørsholm (DK)
(72) Inventor: Goury, Laura, 2970 Hørsholm (DK); Drelon, Marie, 2970 Hørsholm (DK); Mazet, Remi, 2970 Hørsholm (DK)
(74) Representative: Willnegger, Eva

(56) References cited:
- EP-A1- 2 717 720
- GB-A- 2 248 170
- US-A1- 2019 254 302
- US-A1- 2020 178 564

## Description

### FIELD OF THE INVENTION

The present invention relates to a composition for coloring foods, beverages, animal feeds, cosmetics or drugs, in particular the coloring of cheese.

### BACKGROUND OF THE INVENTION

Beta-carotene is a useful cheese coloring agent because it is naturally present in milk. Beta-carotene gives cheese a color which appeals to the customer and it does not produce off-flavors during the ripening of the cheese.

However, beta-carotene preparations face the problem that a large amount of color is lost in the whey during the cheese making process. This loss negatively impacts the suitability of the whey for its further processing to infant nutrition.

WO2010/069889 to Cybercolors relates to a composition for coloring cheese curd comprising a combination of an oil phase comprising an effective amount of paprika, an effective amount of a carotenoid, and a fat, and an aqueous phase comprising a caseinate. However, no native casein is disclosed as first hydrocolloid nor second hydrocolloid.

EP2717720 to Chr. Hansen Natural Colors A/S describes a liquid coloring composition comprising:
a) an oil-in-water emulsion comprising a carotenoid dissolved in an oil phase, wherein the oil phase is emulsified in an aqueous phase using a suitable emulsifier and wherein the oil phase is present in an amount of between 5% w/w to 40% w/w of the oil-in-water emulsion; and
b) a formulation of water dispersible carotenoid particles encapsulated with a second hydrocolloid, wherein the suitable hydrocolloid is present in an amount of at least 0.5% w/w of the formulation;
wherein the ratio of a):b) is between 1:100 to 100:1 and wherein the total carotenoid content of a) and b) is between 0.1% w/w to 15% w/w of the resulting liquid coloring composition. The suitable emulsifier, called first hydrocolloid, preferably is selected from the group consisting of lecithin, caseinate and a combination thereof. However, no native casein is disclosed as first hydrocolloid nor second hydrocolloid.

US20190254302 to Cornell University discloses microcapsules comprising a core comprising a coloring agent such as a carotenoid dispersed in a gel matrix such as 2lginate; a proteolytically-cleavable outer polymer shell surrounding said core; and an enzymatically-cleavable lipid layer such as glycerol between said core and said shell.

GB2248170 to Hoffmann-La Roche describes a beta-carotene emulsion composition for coloring cheese curd comprising beta-carotene, a fat, a caseinate and an aqueous solution of gelatin.

US2020/178564 to Deutsches Institut für Lebensmitteltechnik describes a process in which casein micelles are suspended in an aqueous medium and treted with pulsed electric fields in order to produce casein micelles which contain at least one lipophilic compound as a preparation which is suspended in the aqueous medium.

Thus, there is a need for alternative, beta-carotene coloring compositions perceived as natural that prevent coloration of the whey.

### SUMMARY OF THE INVENTION

The aim of the present invention is to provide an improved pigment composition using a carotenoid for coloring of foods, such as cheeses. Especially, it is an aim of the present invention to provide an improved coloring composition comprising beta-carotene which is capable meeting consumer expectations in respect of natural compounds. Moreover, the present invention aims at preventing the coloration of the whey. Finally, the present invention aims at providing natural solution to calcium caseinate described in EP2717720.

The inventors surprisingly found that native casein can replace casein derivatives such as calcium caseinate whilst providing a very good coloration of cheese and reducing the coloration of the whey.

Accordingly, a first aspect of the invention is a liquid coloring composition comprising:
a) an oil-in-water emulsion comprising a carotenoid dissolved in an oil phase, wherein the oil phase is emulsified in an aqueous phase using a first hydrocolloid and wherein the oil phase is present in an amount of between 5% w/w to 25% w/w of the oil-in-water emulsion and wherein the first hydrocolloid is present in an amount from about 1% w/w to about 20% w/w of the oil-in-water emulsion; and
b) a formulation of water dispersible carotenoid particles encapsulated with a second hydrocolloid, wherein the second hydrocolloid is present in an amount of at least 2% w/w of the formulation;

wherein the ratio of a) : b) is between 1:10 to 10:1;
wherein the total carotenoid content of a) and b) is between 0.1 % w/w to 15 % w/w, preferably between 1 % w/w to 5 % w/w of the resulting liquid coloring composition; and
wherein the first hydrocolloid is native casein.

In one aspect, the first hydrocolloid is native casein and the second hydrocolloid is calcium caseinate. In another aspect of the liquid coloring composition, both the first hydrocolloid and the second hydrocolloid are native casein.

In another aspect, the liquid coloring composition, and in particular oil-in-water emulsion, is substantially free of casein derivatives, caseinate, such as calcium caseinate. In one embodiment, no calcium caseinate is present in the liquid coloring composition in an amount sufficient to serve as first hydrocolloid for the carotenoid.

In another aspect of the liquid coloring composition, the oil phase is present in an amount of between about 10% w/w to about 20% w/w of the oil-in-water emulsion a).

In another aspect of the liquid coloring composition, the ratio of the oil-in-water emulsion a) to the formulation of water dispersible carotenoid particles encapsulated with a second hydrocolloid b) is between about 10:1 to about 1:10, preferably from 1:5 to about 5:1, or from about 4:1 to about 1:4, or from to about 3:1 to about 1:3, or from about 1:2 to about 2:1.

In another aspect, the carotenoid is selected from the group consisting of beta-carotene, alpha-carotene, gamma-carotene, zeaxanthin, lutein, apocarotenal, cis/trans isomers thereof and combinations thereof.

In another aspect, the carotenoid is beta-carotene.

In another aspect, the first hydrocolloid is native casein.

In another aspect, the first hydrocolloid further comprises lecithin, citric acid esters, mono/di-glycerides, poly-oxyethylene sorbitan fatty acid esters, milk proteins, pea proteins, acacia gum and combinations thereof.

In another aspect, the oil phase comprises a fat selected from the group consisting of lard, oils and combinations thereof.

In another aspect of the liquid coloring composition the fat is an oil selected from sunflower seed oil, corn oil, soybean oil, olive oil, coconut oil, palm oil, palm kernel oil, groundnut oil, cottonseed oil, grapeseed oil, rapeseed oil, medium-chain triglyceride oils, and combinations thereof.

In another aspect, the second hydrocolloid is selected from the group consisting of non-modified starch, modified starch, milk protein, native casein, pea protein, beet pectin and combinations thereof.

A further aspect of the present invention is a method for preparing a liquid coloring composition comprising the steps of:
▪ preparing an oil-in-water emulsion of a dissolved carotenoid using a first hydrocolloid by:
   ▪ preparing an aqueous phase;
   ▪ preparing an oil phase by heating a fat comprising the carotenoid to a temperature suitable for dissolving the carotenoid; and
   ▪ mixing the oil phase in the aqueous phase;
   ▪ preparing a formulation of carotenoid particles encapsulated in a second hydrocolloid by milling; and
   ▪ mixing the oil-in-water emulsion of a) with the encapsulated carotenoid particle formulation of b) in a ratio of between 1:10 to 10:1;
   wherein the first hydrocolloid is native casein.

In another aspect, the temperature suitable for dissolving the carotenoid is between about 140°C to 180°C in the oil-in-water emulsion.

A further aspect is a method of coloring cheese comprising the steps of:
▪ adding a starter culture, rennet and the liquid coloring composition according to any of claims 1 to 10 to milk;
▪ incubating the mixture under conditions favorable to the generation of curd and whey fractions; and
▪ separating the curds from the whey.

A further aspect is a food product, a cheese, a beverage, an animal feed, a cosmetic or a drug comprising the liquid coloring composition of the invention.

A further aspect of the invention is the use of the liquid coloring composition of the invention as a colorant for a food product, a cheese, a beverage, an animal feed, a cosmetic or a drug.

### DETAILED DESCRIPTION OF THE INVENTION

### Definitions

The term "pigment" relates to a material that changes the color of light it reflects as the result of selective color absorption. This physical process differs from fluorescence, phosphorescence, and other forms of luminescence, in which the material itself emits light.

The term "carotenoid" as used herein comprises carotenes or structurally related polyene compounds, such as xanthophylls, which can be used as a colorant for foods, beverages, animal feeds, cosmetics or drugs. Examples of such carotenoids are alpha- or beta-carotene, 8'-apo-beta-carotenal, 8'-apo-beta-carotenoic acid esters such as ethyl ester, canthaxanthin, astaxanthin, lycopene, lutein, zeaxanthin or crocetin, or mixtures thereof. As already mentioned, the preferred carotenoid is beta-carotene.

The term "native casein" describes a mixed complex of phosphoproteins isolated from mammal milk and in particular from cow milk as colloidally dispersed micelles from 50 to 600 nanometers in diameter. The variable-sized micelles comprise spherical subunits of 10 to 20 nm, containing 25 to 30 casein molecules. Casein may be separated from the whey proteins of milk for example by gel-filtration or high-speed centrifugation.

The term "casein derivative" describes compounds that are obtained by chemical or structural (other than separation by filtration or centrifugation) modification of native casein, for example acidification. Examples of casein derivatives are caseinate, such as calcium caseinate.

The term "calcium caseinate" describes a derivative of native casein. Calcium caseinate is produced by adding an alkali to acid casein. Calcium caseinate is currently used in stabilizing color formulations in cheese production. However, some of the color pigments may still migrate to the whey. Consequently, the whey becomes less suitable for further processing to powdered milk for infant nutrition. Therefore, it is an object of the invention to reduce this migration of color pigments into the whey.

### Implementation and aspects of the invention

The liquid coloring composition of the present invention is made up of 2 main fractions:
a) an emulsified carotenoid (oil-in-water emulsion) formulation and
b) a formulation with water dispersible carotenoid particles encapsulated with a hydrocolloid.

In a preferred embodiment of the present invention the ratio of a):b) is between about 1:100 to about 100:1, such as between about 1:10 to about 10:1, such as between about 1:5 to about 5:1.

Carotenoids from natural sources as well as their nature-identical counterparts may be used.

In a preferred embodiment of the present invention, the coloring composition is made with the following carotenoids: beta-carotene, alpha-carotene, gamma-carotene, zeaxanthin, lutein, cis/trans isomers and combinations thereof.

In an even more preferred embodiment the carotenoid is beta-carotene.

The first hydrocolloid preferably is native casein. The first hydrocolloid may further comprise lecithin, citric acid esters, mono/di-glycerides, poly-oxyethylene sorbitan fatty acid esters, milk proteins, pea proteins, acacia gum and combinations thereof.

The amount of first hydrocolloid used in the oil-in-water emulsion is from about 1% weight per weight to about 20% weight per weight, such as from about 2% weight per weight to about 15% weight per weight.

The second hydrocolloid preferably is native casein. The second hydrocolloid may further comprise lecithin, citric acid esters, mono/di-glycerides, poly-oxyethylene sorbitan fatty acid esters, milk proteins, pea proteins, acacia gum and combinations thereof.

Ascorbyl palmitate may be added to regenerate antioxidants present at the emulsion interface.

Other ingredients in the water phase may include sugar, starches and organic acids.

Preferably, the oil phase comprises a fat selected from the group consisting of lard, or a vegetable oil, such as sunflower seed oil, corn oil, soybean oil, olive oil, coconut oil, palm oil, palm kernel oil, groundnut oil, cottonseed oil, grapeseed oil, rapeseed oil or medium-chain triglyceride oil, and combinations thereof.

The amount of fat used in the oil-in-water emulsion may be between about 5% weight per weight to about 25% weight per weight, preferably about 10% weight per weight to about 20% weight per weight. Antioxidants, preferable alpha-tocopherol or mixed tocopherols, may be added to the oil phase in order to protect carotenoids during heating and subsequently stabilize the final color formulation against oxidative degradation.

Antimicrobials such as organic acids or salts thereof, e.g. sorbic acid, benzoic acid, may be used to inhibit growth of microorganisms during storage.

Preferably, the composition further comprises an emulsion carrier. Suitably, the emulsion carrier may be selected from the group consisting of maltodextrin, sucrose, glucose, fructose, lactose, maltose, invert sugar, and combinations thereof.

The carotenoid particles may comprise amorph carotenoid as well as crystalline carotenoid. Preferably, the carotenoid particles comprise 100% crystalline carotenoid.

In a preferred embodiment the amount of hydrocolloid in the milled fraction is at least about 0.5% weight per weight, such as at least about 2% weight per weight.

Preferably, the total beta-carotene pigment content is from about 0.1% weight per weight to about 15% weight per weight, more preferably from about 0.5% weight per weight to about 10% weight per weight.

The process of preparing the coloring composition comprises three steps: An oil-in-water emulsion of a dissolved carotenoid is prepared using a first hydrocolloid by i) preparing an aqueous phase; ii) preparing an oil phase by heating a fat comprising the carotenoid to a temperature suitable for dissolving the carotenoid; and iii) mixing the oil phase in the aqueous phase by stirring;

The term "a temperature suitable for dissolving the carotenoid" refers to a temperature in a range from about 120°C to about 230°C, at which temperature the carotenoid can be dissolved in the fat during the process of preparing the oil-in-water emulsion.

In a preferred embodiment, the temperature suitable for dissolving the carotenoid is between about 140°C to about 180°C, preferably form about 150°C to about 180°C.

The two phases are mixed using high speed propeller stirring, and the subsequent processing step is an emulsification by homogenization. A particle size distribution with an average particle size in the range 0.25 - 2.50 µm is obtained following the emulsification of this fraction.

A fraction containing water-dispersible, encapsulated particles of carotenoid is produced by milling the carotenoid particles either in dry form or as a water suspension with a hydrocolloid, such as native casein, non-modified starch or modified starch, milk or pea protein or beet pectin, thereby stabilizing the suspension of the encapsulated carotenoid in aqueous phase and avoiding slow dissolution of particles in presence of oily phases. Moreover, the colorimetric properties may be improved in the cheese application.

The suspension is heated moderately, e.g. to 55°C, while mixing by high speed propeller stirring and a pre-emulsification. The suspension is thereafter submitted to ball mill step in order to increase the surface area of the particles while simultaneously ensuring coverage of particles with stabilizing hydrocolloid.

The final step is to mix the emulsion fraction and the milled fraction by stirring. It is also possible to mix ratios that ensure a desired reddish shade in the final application.

As mentioned above, an aspect of the present invention relates to a food product, a beverage, an animal feed, a cosmetic or a drug comprising the coloring composition according to the present invention.

In a preferred embodiment, the food product is a cheese, such as a traditional cheese or a processed cheese.

The coloring composition of the present invention may find use as a colorant for cheese, such as traditional cheese or processed cheese.

The coloring composition of the present invention may find use as colorant not only for cheese but also for other foods, beverages, animal feeds, cosmetic and drugs.

As will be appreciated by the skilled person, the coloring composition of the present invention may be added either as an aqueous stock solution or a pre-blend with other suitable food ingredients according to the specific application. Mixing may be carried out e.g. using a low shear mixer, a high pressure homogenizer or a high shear mixer depending on the formulation of the final application. Mixing procedure and amount of oily or aqueous ingredients may impact the color of the final application. As will be readily apparent such technicalities are within the skill of the expert.

Embodiments of the present invention are described below, by way of non-limiting examples.

### EXAMPLES

### Process

### Emulsified fraction:

The emulsified part is processed with a homogenizer APV lab 1000 from SPX.

All emulsions were prepared with the following order of addition and process:
1) Prepare the aqueous phase by adding water, native casein.
2) Prepare the oil phase by adding oil, carotene. Heat until a fully dissolution of pigment.
3) Transfer the oil phase into the water phase
4) Homogenize
5) Pasteurize

### Milled fraction:

The milled fraction is milled with a ball mill from WAB.

All encapsulations were prepared with the following order of addition and process:
1) Prepare the water phase by adding water, and native casein.
2) Add carotene
4) Pasteurize
5) Mill the suspension on a ball mill

As a final step, a simple mixing of the emulsion fraction and the milled fraction by stirring while adding ratios that ensure the desired reddish shade in the final application.

### Analysis

The Malvern Zetasizer Nano-S is used to determine the Z-average (Zave) particle size and PDI (polydispersity index) when the size is from 1nm to 1 µm. One drop of the liquid color is added in 40 mL of demineralized water. This solution is put in the Zetasizer. A laser passes through the solution and the intensity fluctuations due to Brownian movements is measured and converted to size.

The pigment contents in the two parts and blend are determined with a UV-spectrophotometer UV-1800 from Shimadzu by dissolving the formulation in water and then acetone at appropriate dilution. The extinction coefficient used to determine the concentration of beta-carotene (w w %) is 2559 at a λ = 452 nm.

The lightness, chroma and hue are obtained with a Datacolor 650 in reflexion. All samples are analyzed in milk 3.5%fat.

### Lab Cheese

### Color Evaluations

Color measurements are performed on the colorimeter Probe4Light The Cheese was produced as follows. For 200 g of milk following compounds were added in following order while stirring and following process steps were carried:
1) Add Culture: 4g of pre-blend of 2.5 g culture to 125 g milk
2) Add CaCl2: 4 drops of 10% CaCl2
3) Fermentation for 45 minutes at 32°C
4) Add 1 wt% or less, preferably 0,1 wt% or less, here 0,1 w% of liquid color composition, 15 minutes before end of fermentation time.
5) Add rennet: 400 microliters pre-blend produced by adding 1000 microliters to 7 g water.
6) Renneting for 45 minutes at 32°C.
7) Cutting
8) Pre-stirring for 15 minutes
9) Scalding for 15 minutes at 40°C with slow stirring
10) Final stirring for 7 minutes
11) Whey off

### Example 1

To determine the content of native casein suitable for our purpose, different emulsions and encapsulations were performed containing water and native casein at different ratios. Then, in cheese application, they were compared to the reference of each part (emulsified or milled) based on calcium caseinate.

### Formulation recipes:

All recipes were processed following the different steps as described above. The process was stopped when expected shade and particle size were reached.

| | Emulsified part | | Milled part | |
|---|---|---|---|---|
| | Ref 1 | A | Ref 2 | B |
| | % wt | % wt | %wt | % wt |
| Liquid Carotenoid | 7,55 | 7,55 | | - |
| Crystals of carotenoid | - | - | 2,1 | 2.1 |
| Sunflower oil | 6,30 | 6,30 | | - |
| Water | 36,90 | 36,90 | 36,4 | 37.2 |
| Other ingredients | 42.5 | 42.5 | 54,3 | 55.7 |
| Native casein | | **6,75** | | **5** |
| Calcium caseinate | 6.75 | | 7,2 | |
| ***Total*** | 100 | 100 | 100 | 100 |

All ratios of native casein in the emulsified and the milled portions have led to a liquid bulk similar to the references. Surprisingly, it was possible to increase and decrease the level of casein without interfering with the viscosity and stabilization.

| **Trial** | **Strength (%)** | **L** | **C** | **h** | **Zave (nm)** | **PDi** |
|---|---|---|---|---|---|---|
| Ref 1 | 1.9-2.1 | 80-85 | 40-50 | 70-75 | < 550 | < 0.4 |
| A | 1.9-2.1 | 80-85 | 40-50 | 70-75 | < 550 | < 0.4 |
| Ref 2 | 1.9-2.1 | 75-85 | 15-25 | 25-35 | < 550 | < 0,4 |
| B | 1.9-2.1 | 75-85 | 15-25 | 25-35 | < 550 | < 0.4 |

### Application test and whey analysis:

| **Trial** | **L** | **a** | **b** | **C** | **h** | **DE2000** |
|---|---|---|---|---|---|---|
| Ref 1 | 90 | -6 | 43 | 43 | 99 | - |
| A | 91 | -8 | 39 | 40 | 102 | 2 |
| Ref 2 | 93 | -8 | 32 | 33 | 104 | - |
| B | 91 | -6 | 33 | 34 | 101 | 1.6 |

All formulations based on native casein surprisingly exhibit less beta-carotene color into the whey. Indeed, the chroma value of sample A is lower than the reference for emulsified product and hue is higher compared to the reference. Consequently, a lower amount of pigment has migrated from the curd into the whey resulting in a whiter whey.

Regarding the milled fraction where there is native casein, there is the same coloration and whey performance as calcium caseinate whilst being more acceptable to consumers that are interested in natural solutions.

### Example 2

A blend between the emulsified and milled parts was, therefore, produced at a fraction 55/45, respectively based on example 1.

### Formulation recipes:

| **Trial** | **Ref** | **1** |
|---|---|---|
| | % wt | % wt |
| Ref 1 | 55 | |
| Ref 2 | 45 | |
| A | | 55 |
| B | | 45 |
| Total | 100 | 100 |

| **Trial** | **Strength (%)** | **L** | **C** | **h** | **Zave (nm)** | **PDi** |
|---|---|---|---|---|---|---|
| Ref | 1.9-2.1 | 75-85 | 30-40 | 60-65 | < 550 | < 0.4 |
| 1 | 1.9-2.1 | 75-85 | 30-40 | 60-65 | < 550 | < 0.4 |

### Application test and whey analysis:

The blend was compared to the reference based on calcium caseinate and same ratios between emulsified and milled parts.

| **Trial** | **L** | **a** | **b** | **C** | **h** | **DE2000** |
|---|---|---|---|---|---|---|
| Ref | 92 | -7 | 39 | 40 | 100 | - |
| 1 | 93 | -8 | 36 | 37 | 106 | 1.7 |

Surprisingly, a reduction of beta-carotene transfer was observed in the blend containing native casein compared to the reference based on calcium caseinate. The emulsified part has an unexpected synergic effect with the milled portion leading, when blended, to a significant decrease of pigment migration from the curd to the whey.

### Example 3: Addition of hydrocolloids in emulsified part

A hydrocolloid was added to the emulsified part in recipes containing native casein. All recipes were processed following the different steps as described above. Process was stopped when good shade and particle size were reached.

### Formulation recipes:

| | Ref 1 | Ref 2 | A | B |
|---|---|---|---|---|
| | % wt | % wt | % wt | % wt |
| Liquid carotenoid | 7,55 | 7,55 | 7,55 | 7,55 |
| Sunflower oil | 6.30 | 6.30 | 6,30 | 6,30 |
| Water | 36.90 | 36.90 | 36.9 | 36,90 |
| Other ingredients | 42.5 | 42.5 | 41.75 | 41.75 |
| Native casein | | 6.75 | 6.75 | 6.75 |
| Calcium caseinate | 6.75 | | | |
| Gum Arabic | | | | **0.75** |
| Modified starch | | | **0.75** | |
| ***Total*** | 100 | 100 | 100 | 100 |

| **Trial** | **Strength (%)** | **L** | **C** | **h** | **Zave (nm)** | **PDi** |
|---|---|---|---|---|---|---|
| Ref 1 | 1.9-2.1 | 80-85 | 40-50 | 70-75 | < 550 | < 0.4 |
| Ref 2 | 1.9-2.1 | 80-85 | 40-50 | 70-75 | < 550 | < 0.4 |
| A | 1.9-2.1 | 80-85 | 40-50 | 70-75 | < 550 | < 0.4 |
| B | 1.9-2.1 | 80-85 | 40-50 | 70-75 | < 550 | < 0.4 |

### Application test and whey analysis:

The addition of a hydrocolloid has, surprisingly, lead to a better retention of the beta-carotene in the curd. Indeed, the transfer of pigment is lower when a hydrocolloid is added. The chroma value of samples A and B is lower than the reference for emulsified product and hue is higher compared to the reference. The native casein and the additional hydrocolloid have a synergic effect leading to an unexpected better stabilization of the pigment.

| **Trial** | **L** | **a** | **b** | **C** | **h** | **DE2000** |
|---|---|---|---|---|---|---|
| Ref 1 | 91 | -7 | 42 | 43 | 99 | - |
| Ref 2 | 92 | -7 | 39 | 40 | 100 | 1.23 |
| A | 92 | -8 | 35 | 36 | 102 | 2.81 |
| B | 92 | -7 | 37 | 37 | 101 | 2.27 |

### Comparative example 1: Use of emulsifier in emulsified part

An emulsifier was used instead of native casein to stabilize the emulsified part. All recipes were processed following the different steps as described above. Process was stopped when good shade and particle size were reached.

### Formulation recipes:

| | Ref 1 | A |
|---|---|---|
| | % wt | % wt |
| Liquid carotenoid | 7,55 | 7,55 |
| Sunflower oil | 6.30 | 21.05 |
| Water | 36.90 | 37.90 |
| Other ingredients | 42.5 | 34.2 |

| | | |
|---|---|---|
| Calcium caseinate | 6.75 | |
| Citrem | | 2.00 |
| ***Total*** | 100 | 100 |

| **Trial** | **Strength (%)** | **L** | **C** | **h** | **Zave (nm)** | **PDi** |
|---|---|---|---|---|---|---|
| Ref 1 | 1.9-2.1 | 80-85 | 40-50 | 70-75 | < 550 | < 0.4 |
| A | 1.9-2.1 | 80-85 | 40-50 | 70-75 | < 550 | < 0.4 |

### Application test and whey analysis:

The use of citrem instead of casein leads to a high migration of beta-carotene from the curd to the whey. An unexpected very intense whey was observed with an important chroma due to a significant pigment transfer.

| **Trial** | **L** | **a** | **b** | **C** | **h** | **DE2000** |
|---|---|---|---|---|---|---|
| Ref | 90 | -5 | 42 | 42 | 97 | - |
| A | 87 | -2 | 64 | 64 | 92 | 7 |

### Comparative example 2: Use of a hydrocolloid in emulsified part

An hydrocolloid was used instead of native casein to stabilize the emulsified part. All recipes were processed following the different steps above. Process was stopped when expected shade and particle size were reached.

### Formulation recipes:

| | Ref | B |
|---|---|---|
| | % wt | % wt |
| Liquid carotenoid | 7,55 | |
| Crystals of carotenoid | | 2.5 |
| Sunflower oil | 6.30 | 4.00 |
| Water | 36.90 | 44.00 |
| Other ingredients | 42,5 | 29,5 |
| Calcium caseinate | 6.75 | |
| Gum Arabic | | 20.00 |
| ***Total*** | 100 | 100 |

| **Trial** | **Strength (%)** | **L** | **C** | **h** | **Zave (nm)** | **PDi** |
|---|---|---|---|---|---|---|
| Ref | 1,9-2,1 | 80-85 | 40-50 | 70-75 | < 550 | < 0.4 |
| B | 1,9-2,1 | 80-85 | 40-50 | 70-75 | < 550 | < 0.4 |

### Application test and whey analysis:

Gum arabic instead of native casein did not retain the beta-carotene in the cheese curd and by consequence, highly colored the whey. A very high chroma and low hue were obtained demonstrating an orange shade of the whey due to a significant transfer of pigment.

| **Trial** | **L** | **a** | **b** | **C** | **h** | **DE2000** |
|---|---|---|---|---|---|---|
| Ref | 90 | -5 | 42 | 42 | 97 | - |
| A | 69 | 35 | 111 | 117 | 72 | 27.9 |

## Claims

1. A liquid coloring composition comprising:
a) an oil-in-water emulsion comprising a carotenoid dissolved in an oil phase, wherein the oil phase is emulsified in an aqueous phase using a first hydrocolloid and wherein the oil phase is present in an amount of between 5% w/w to 25% w/w of the oil-in-water emulsion and wherein the first hydrocolloid is present in amount from about 1% w/w to about 20% w/w of the oil-in-water emulsion; and
b) a formulation of water dispersible carotenoid particles encapsulated with a second hydrocolloid, wherein the second hydrocolloid is present in an amount of at least 2% w/w of the formulation;
wherein the ratio of a):b) is between 1:10 to 10:1;
wherein the total carotenoid content of a) and b) is between 0.1 % w/w to 15 % w/w, preferably between 1% w/w to 5 % w/w of the resulting liquid coloring composition; and
wherein the first hydrocolloid is native casein.

2. The liquid coloring composition according to any of the preceding claims, wherein the oil phase is present in an amount of between about 10% w/w to about 20% w/w of the oil-in-water emulsion a).

3. The liquid coloring composition according to any of the preceding claims, wherein the ratio of the oil-in-water emulsion a) to the formulation of water dispersible carotenoid particles encapsulated with a second hydrocolloid b) is between about 1:5 to about 5:1.

4. The liquid coloring composition according to any of the preceding claims, wherein the carotenoid is selected from the group consisting of beta-carotene, alpha-carotene, gamma-carotene, zeaxanthin, lutein, cis/trans isomers thereof and combinations thereof.

5. The liquid coloring composition according to any of the preceding claims, wherein the carotenoid is beta-carotene.

6. The liquid coloring composition according to any of the preceding claims, wherein the first hydrocolloid further comprises lecithin, citric acid esters, mono/di-glycerides, poly-oxyethylene sorbitan fatty acid esters, milk proteins, pea proteins, acacia gum and combinations thereof.

7. The liquid coloring composition according to any of the preceding claims, wherein the oil phase comprises a vegetable oil selected from sunflower seed oil, corn oil, soybean oil, olive oil, coconut oil, palm oil, palm kernel oil, groundnut oil, cottonseed oil, grape seed oil, rape seed oil, medium-chain triglyceride oils, and combinations thereof.

8. The liquid coloring composition according to any of the preceding claims, wherein the second hydrocolloid is selected from the group consisting of non-modified starch, modified starch, gum Arabic, milk protein, native casein, pea protein, beet pectin and combinations thereof.

9. A method for preparing a liquid coloring composition comprising the steps of:
a) preparing an oil-in-water emulsion of a dissolved carotenoid using a first hydrocolloid by:
i) preparing an aqueous phase;
ii) preparing an oil phase by heating a fat comprising the carotenoid to a temperature suitable for dissolving the carotenoid; and
iii) mixing the oil phase in the aqueous phase;
b) preparing a formulation of carotenoid particles encapsulated in a second hydrocolloid by milling process; and
c) mixing the oil-in-water emulsion of a) with the encapsulated carotenoid particle formulation of b) in a ratio of between 1:10 to 10:1;
wherein the first hydrocolloid is native casein.

10. A food product, a cheese, a beverage, an animal feed, a cosmetic or a drug comprising the liquid coloring composition according to any of claims 1 to 8 or a liquid coloring composition obtained according to claim 9.

11. Use of the liquid coloring composition according to any of claims 1 to 8 or obtained according to claim 9 as a colorant for a food product, a cheese, a beverage, an animal feed, a cosmetic or a drug.

## Patentansprüche

1. Eine flüssige Farbzusammensetzung, die Folgendes umfasst:
a) eine Öl-in-Wasser-Emulsion, die ein in einer Ölphase gelöstes Carotinoid umfasst, wobei die Ölphase in einer wässrigen Phase unter Verwendung eines ersten Hydrokolloids emulgiert ist und wobei die Ölphase in einer Menge von zwischen 5 Gew.-% bis 25 Gew.-% der Öl-in-Wasser-Emulsion vorhanden ist und wobei das erste Hydrokolloid in einer Menge von etwa 1 Gew.-% bis etwa 20 Gew.-% der Öl-in-Wasser-Emulsion vorhanden ist; und
b) eine Formulierung aus in Wasser dispergierbaren Carotinoidteilchen, die mit einem zweiten Hydrokolloid eingekapselt sind, wobei das zweite Hydrokolloid in einer Menge von mindestens 2 Gew.-% der Formulierung vorhanden ist;
wobei das Verhältnis von a):b) zwischen 1:10 und 10:1 liegt;
wobei der Gesamtcarotinoidgehalt von a) und b) zwischen 0,1 Gew.-% bis 15 Gew.-%, vorzugsweise zwischen 1 Gew.-% bis 5 Gew.-% der resultierenden flüssigen Farbzusammensetzung liegt; und
wobei das erste Hydrokolloid natives Kasein ist.

2. Flüssige Farbzusammensetzung nach einem der vorangehenden Ansprüche, wobei die Ölphase in einer Menge von etwa 10 Gew.-% bis etwa 20 Gew.-% der Öl-in-Wasser-Emulsion a) vorhanden ist.

3. Flüssige Farbzusammensetzung nach einem der vorhergehenden Ansprüche, wobei das Verhältnis der Öl-in-Wasser-Emulsion a) zu der Formulierung von wasserdispergierbaren Carotinoidteilchen, die mit einem zweiten Hydrokolloid b) eingekapselt sind, zwischen etwa 1:5 und etwa 5:1 liegt.

4. Flüssige Farbzusammensetzung nach einem der vorhergehenden Ansprüche, wobei das Carotinoid ausgewählt ist aus der Gruppe bestehend aus Beta-Carotin, Alpha-Carotin, Gamma-Carotin, Zeaxanthin, Lutein, cis/trans-Isomeren davon und Kombinationen davon.

5. Flüssige Farbzusammensetzung nach einem der vorhergehenden Ansprüche, wobei das Carotinoid Beta-Carotin ist.

6. Flüssige Farbzusammensetzung nach einem der vorhergehenden Ansprüche, wobei das erste Hydrokolloid weiterhin Lecithin, Zitronensäureester, Mono-/DiGlyceride, Polyoxyethylensorbitanfettsäureester, Milchproteine, Erbsenproteine, Akaziengummi und Kombinationen davon umfasst.

7. Flüssige Farbzusammensetzung nach einem der vorhergehenden Ansprüche, wobei die Ölphase ein Pflanzenöl umfasst, das aus Sonnenblumenkernöl, Maisöl, Sojabohnenöl, Olivenöl, Kokosnussöl, Palmöl, Palmkernöl, Erdnussöl, Baumwollsamenöl, Traubenkernöl, Rapsöl, mittelkettigen Triglyceridölen und Kombinationen davon ausgewählt ist.

8. Flüssige Farbzusammensetzung nach einem der vorhergehenden Ansprüche, wobei das zweite Hydrokolloid aus der Gruppe ausgewählt ist, die aus nicht modifizierter Stärke, modifizierter Stärke, Gummi Arabicum, Milchprotein, nativem Kasein, Erbsenprotein, Rübenpektin und Kombinationen davon besteht.

9. Verfahren zur Herstellung einer flüssigen Farbzusammensetzung, das die folgenden Schritte umfasst:
a) Herstellung einer Öl-in-Wasser-Emulsion eines gelösten Carotinoids unter Verwendung eines ersten Hydrokolloids durch:
i) Herstellung einer wässrigen Phase;
ii) Herstellung einer Ölphase durch Erhitzen eines Fetts, das das Carotinoid enthält, auf eine zum Lösen des Carotinoids geeignete Temperatur; und
iii) Mischen der Ölphase mit der wässrigen Phase;
b) Herstellung einer Formulierung von Carotinoidteilchen, die in einem zweiten Hydrokolloid eingekapselt sind, durch ein Mahlverfahren; und
c) Mischen der Öl-in-Wasser-Emulsion von a) mit der eingekapselten Carotinoidpartikelformulierung von b) in einem Verhältnis von 1:10 bis 10:1;
wobei das erste Hydrokolloid natives Kasein ist.

10. Lebensmittelprodukt, Käse, Getränk, Tierfutter, Kosmetikum oder Arzneimittel, das die flüssige Farbzusammensetzung nach einem der Ansprüche 1 bis 8 oder eine nach Anspruch 9 erhaltene flüssige Farbzusammensetzung enthält.

11. Verwendung der flüssigen Farbzusammensetzung nach einem der Ansprüche 1 bis 8 oder erhalten nach Anspruch 9 als Färbemittel für ein Lebensmittelprodukt, einen Käse, ein Getränk, ein Tierfutter, ein Kosmetikum oder ein Arzneimittel.

## Revendications

1. Composition colorante liquide comprenant
a) une émulsion huile dans eau comprenant un caroténoïde dissous dans une phase huileuse, dans laquelle la phase huileuse est émulsionnée dans une phase aqueuse à l'aide d'un premier hydrocolloïde et dans laquelle la phase huileuse est présente en une quantité comprise entre 5 % p/p et 25 % p/p de l'émulsion huile dans eau et dans laquelle le premier hydrocolloïde est présent en une quantité comprise entre environ 1 % p/p et environ 20 % p/p de l'émulsion huile dans eau ; et
b) une formulation de particules de caroténoïdes dispersables dans l'eau, encapsulées dans un second hydrocolloïde, dans laquelle le second hydrocolloïde est présent à hauteur d'au moins 2 % p/p de la formulation ;
dans lequel le rapport a):b) est compris entre 1:10 et 10:1 ;
dans laquelle la teneur totale en caroténoïdes des points a) et b) est comprise entre 0,1 % p/p et 15 % p/p, de préférence entre 1 % p/p et 5 % p/p de la composition colorante liquide résultante ; et
dans lequel le premier hydrocolloïde est de la caséine native.

2. La composition colorante liquide selon l'une des revendications précédentes, dans laquelle la phase huileuse est présente dans une quantité comprise entre environ 10 % p/p et environ 20 % p/p de l'émulsion huile dans eau a).

3. La composition colorante liquide selon l'une des revendications précédentes, dans laquelle le rapport entre l'émulsion huile dans eau a) et la formulation de particules de caroténoïdes dispersables dans l'eau encapsulées avec un second hydrocolloïde b) est compris entre environ 1:5 et environ 5:1.

4. La composition colorante liquide selon l'une des revendications précédentes, dans laquelle le caroténoïde est choisi dans le groupe constitué du bêta-carotène, de l'alpha-carotène, du gamma-carotène, de la zéaxanthine, de la lutéine, de leurs isomères cis/trans et de leurs combinaisons.

5. La Composition colorante liquide selon l'une des revendications précédentes, dans laquelle le caroténoïde est le bêta-carotène.

6. La Composition liquide colorante selon l'une des revendications précédentes, dans laquelle le premier hydrocolloïde comprend en outre de la lécithine, des esters d'acide citrique, des mono/di-glycérides, des esters d'acide gras de poly-oxyéthylène sorbitane, des protéines de lait, des protéines de pois, de la gomme d'acacia et des combinaisons de ceux-ci.

7. La composition liquide colorante selon l'une quelconque des revendications précédentes, dans laquelle la phase huileuse comprend une huile végétale choisie parmi les huiles de graines de tournesol, de maïs, de soja, d'olive, de noix de coco, de palme, de palmiste, d'arachide, de coton, de pépins de raisin, de colza, les huiles de triglycérides à chaîne moyenne, et leurs combinaisons.

8. La composition liquide colorante selon l'une quelconque des revendications précédentes, dans laquelle le second hydrocolloïde est choisi dans le groupe constitué par l'amidon non modifié, l'amidon modifié, la gomme arabique, la protéine de lait, la caséine native, la protéine de pois, la pectine de betterave et leurs combinaisons.

9. Méthode de préparation d'une composition colorante liquide comprenant les étapes suivantes :
a) préparer une émulsion huile dans eau d'un caroténoïde dissous à l'aide d'un premier hydrocolloïde :
i) la préparation d'une phase aqueuse ;
ii) préparer une phase huileuse en chauffant une matière grasse contenant le caroténoïde à une température permettant de dissoudre le caroténoïde ; et
iii) en mélangeant la phase huileuse à la phase aqueuse ;
b) préparer une formulation de particules de caroténoïdes encapsulées dans un second hydrocolloïde par un procédé de broyage ; et
c) mélanger l'émulsion huile dans eau de a) avec la formulation de particules de caroténoïdes encapsulés de b) dans un rapport compris entre 1:10 et 10:1 ;
dans lequel le premier hydrocolloïde est de la caséine native.

10. Produit alimentaire, fromage, boisson, aliment pour animaux, cosmétique ou médicament comprenant la composition liquide colorante selon l'une quelconque des revendications 1 à 8 ou une composition liquide colorante obtenue selon la revendication 9.

11. Utilisation de la composition colorante liquide selon l'une des revendications 1 à 8 ou obtenue selon la revendication 9 comme colorant pour un produit alimentaire, un fromage, une boisson, un aliment pour animaux, un cosmétique ou un médicament.
